# EUROPÄISCHE PATENTANMELDUNG

(11) **EP 3 854 867 A1**
(43) Veröffentlichungstag der Anmeldung: **28.07.2021**
(21) Anmeldenummer: 20153266.0
(22) Anmeldetag: 23.01.2020
(51) Int. Cl.: C12M 1/00

(54) **VORKEHRUNG ZUR KULTIVIERUNG VON PROKRYOTISCHEN UND EUKARYOTISCHEN ZELLEN IN EINER STRUKTURIERTEN MATRIX UNTER PHYSIKALISCHER KRÄFTEEINWIRKUNG**

(71) Anmelder: FRAUNHOFER-GESELLSCHAFT zur Förderung der angewandten Forschung e.V., 80686 München (DE)
(72) Erfinder: Vogel, Simon, 52074 Aachen (DE); Schillberg, Stefan, 52074 Aachen (DE)
(74) Vertreter: Roth, Andy Stefan

(57) **Zusammenfassung**

Beschrieben wird ein Bioreaktor (1) zur Kultivierung von Zellen mit wenigstens einem Gefäß (2), das eingerichtet ist, um in einem wenigstens teilweise von Wänden (6) des Gefäßes (2) gegenüber einer Umgebung abgeschlossenen Gefäßinnenraum (3) die zu kultivierenden Zellen und zumindest ein Nährmedium aufzunehmen, und mit einer Bewegungseinrichtung (4), die mit einer Antriebseinheit (5) verbindbar ist und die die zu kultivierenden Zellen wenigstens zeitweise bewegt oder mit einer Kraft beaufschlagt

Die beschriebene Lösung zeichnet sich dadurch aus, dass in dem Gefäßinnenraum (3) ein elastisches Element (7) angeordnet ist, das derart ausgeführt ist, dass zumindest in einem Bereich des elastischen Elements wenigstens ein Teil des Nährmediums aufnehmbar ist und dass die zu kultivierenden Zellen wenigstens bereichsweise in und/oder an dem elastischen Element zumindest zeitweise haften und dass die Bewegungseinrichtung (4) eingerichtet ist, um das elastische Element (7) wenigstens zeitweise zu verformen.

## Beschreibung

Die Erfindung betrifft einen Bioreaktor zur Kultivierung von Zellen. Der beschriebene Bioreaktor verfügt über wenigstens ein Gefäß, in dem die zu kultivierenden Zellen und zumindest ein Nährmedium angeordnet sind und über eine Bewegungseinrichtung, die mit einer Antriebseinheit verbindbar ist und die die zu kultivierenden Zellen wenigstens zeitweise bewegt oder mit einer Kraft beaufschlagt

Als Zellkultur wird die Kultivierung tierischer oder pflanzlicher Zellen in einem Nährmedium außerhalb des Organismus bezeichnet Derartige Zellkulturen können Zellen einer Gewebeart unbegrenzt fortpflanzen, wobei sowohl immortalisierte, also unsterbliche Zelllinien, als auch primäre Zellen kultiviert werden. Bei der Kultivierung von Zellen prokaryotischen oder eukaryotischen Ursprungs ist die Schaffung der für den jeweiligen Zelltyp angepassten physiologischen Parameter, die zumindest weitgehend den jeweiligen normalen Lebensvorgängen entsprechen müssen, von entscheidender Bedeutung. Die Festlegung geeigneter physiologischer Parameter ist wichtig, um ein optimales Wachstumsverhalten, also beispielsweise die Ausbildung eines Zellverbandes oder der gewünschten Zellform, eine hohe Wachstumsgeschwindigkeit oder aber die Produktion ausgewählter Inhaltsstoffe zu erreichen. So wird beispielsweise bei der Produktion von Antibiotika häufig mit Biofilmen, also Zellverbänden, die an der Oberfläche von Kulturgefäßen haften, gearbeitet, um so den Stoffwechselweg der Zellen zu modulieren und die Antibiotikaproduktion zu stimulieren.

Im Weiteren ist es bei der Kultivierung von Zellen, insbesondere von Säugetierzellen, bekannt, dass für spezialisierte Zelltypen elektrische oder mechanische Reize oder auch zelltypspezifische Strukturen benötigt werden, um ein gewünschtes Wachstum zu induzieren. So werden etwa elektrische oder physikalische Stimuli benötigt, um Myocardioblasten bzw. Muskelzellen auszubilden, die daraufhin spindelförmige Zellen ausbilden, die sich zu Muskelfasern zusammenlagern.

Zur Kultivierung von Zellen prokaryotischen oder eukaryotischen Ursprungs werden bislang unterschiedliche Kulturgefäße, die auch als Bioreaktoren bezeichnet werden, verwendet, in denen die Zellen unter aeroben oder anaeroben Bedingungen kultiviert werden. Bekannte Bioreaktorsysteme sind Schüttelkulturen oder Fermenter, in denen die Zellen unter Begasung und Rühren oder Schütteln in einer Nährlösung mit Sauerstoff und Nährstoffen versorgt werden. Die Kultivierung der Zellen erfolgt hierbei in der Regel in Suspension.

Durch Zugabe von Trägermaterialien können große Oberflächen für die Bildung eines Films eingesetzt werden.

Eine weitere häufig eingesetzte Form der Zellkultivierung verwendet Festbettreaktoren, in denen eine Zellsuspension durch eine feste Schüttung oder Packung strömt Dieser Prozess wird vor allem industriell genutzt, um etwa Zellen auf einer Oberflächenmatrix zu vermehren und sie zur Ausscheidung von Stoffwechselprodukten anzuregen.

Ein Bioreaktor, der zur Kultivierung von Mikroorganismen, Zellen oder zellfreien Expressionssystemen verwendet wird, ist aus der WO 03/012027 A1 bekannt. In dem beschriebenen Bioreaktor werden biologische oder biochemische Reaktion durchgeführt, die auf dem Gebiet der Biotechnologie, Lebensmitteltechnik und Umweltschutz genutzt werden. Der in dieser Druckschrift beschriebene Bioreaktor verfügt über eine Mehrzahl von Öffnungen, die vor allem zur Zufuhr von Gasen oder Flüssigkeiten, zur Probenentnahme und zur Zufuhr von Mikroorganismen oder Zellen genutzt werden, wobei die entsprechenden Öffnungen mithilfe geeigneter Verschlussmittel verschließbar sind. Ferner verfügt der beschriebene Bioreaktor über einen auffaltbaren oder aufblasbaren Einsatz, der eine effiziente und turbulente Durchmischung der Reaktionsflüssigkeiten ermöglichen soll. Hierbei sind spezielle Schikanen vorgesehen, um während einer Kreisbewegung des Einsatzes und der hierdurch verursachten Bewegung der Reaktionsflüssigkeiten eine effektive Begasung zu gewährleisten.

Ausgehend von den aus dem Stand der Technik bekannten Bioreaktoren liegt der Erfindung die Aufgabe zu Grunde, ein derartiges Kulturgefäß derart weiterzubilden, dass aufgrund einer geeigneten Initiierung von Reizen eine hohe Ausbeute bei der Zellkultivierung erreicht werden kann. Insbesondere soll es möglich sein, spezielle Zellverbände, wie etwa Muskelfasern, die die Applikation externer Stimuli erfordern, in vergleichsweise großem Maßstab anzuziehen und auf diese Weise auch die Herstellung von dreidimensionalen Gewebeverbänden zu ermöglichen. Ein entsprechender Bioreaktor soll des Weiteren geeignet sein, um Muskelfasern als Ausgangsmaterial für die Erzeugung von artifiziellem, kultiviertem Fleisch zu verwenden. Des Weiteren sollte die anzugebende Vorrichtung, also der Bioreaktor, den Bedarf an vergleichsweise einfach aufgebauten und hinsichtlich der erzielbaren Zellausbeute skalierbaren oder parallelisierbaren Apparaturen, in denen Zellen angezogen und die benötigten physikalischen Parameter bedarfsgerecht appliziert werden können, decken können. Vor allem sollte der anzugebende Bioreaktor hierfür unter Einsatz verhältnismäßig einfacher Konstruktionsmittel und unter Verwendung bekannter Bauelemente realisierbar sein, so dass auch unter Berücksichtigung wirtschaftlicher Randbedingungen die Anzucht vergleichsweise großer Zellverbände ermöglicht wird.

Die zuvor genannte Aufgabe wird mit einem Bioreaktor gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert

Ein erfindungsgemäß ausgeführter Bioreaktor zur Kultivierung von Zellen verfügt über wenigstens ein Gefäß, das für die Aufnahme der zu kultivierenden Zellen und zumindest eines Nährmediums geeignet ist, und über eine Bewegungseinrichtung, die mit einer Antriebseinheit verbindbar ist und durch die die zu kultivierenden Zellen wenigstens zeitweise bewegbar oder mit einer Kraft beaufschlagbar sind. Gemäß der Erfindung zeichnet sich der Bioreaktor dadurch aus, dass in einem wenigstens teilweise von Wänden des Gefäßes gegenüber einer Umgebung abgeschlossenen Gefäßinnenraum ein elastisches Element angeordnet ist, das derart ausgeführt ist, das es zumindest teilweise und wenigstens für eine gewisse Zeitspanne Nährmedium aufnehmen kann und die zu kultivierenden Zellen wenigstens bereichsweise in und oder an dem elastischen Element zumindest zeitweise haften und dass die Bewegungseinrichtung eingerichtet ist, um das elastische Element wenigstens zeitweise zu verformen, insbesondere wenigstens bereichsweise zu komprimieren.

Wesentlich für die Erfindung ist somit ein elastisches Element, das wenigstens zeitweise Nährmedium aufnehmen und mithilfe wenigstens einer gezielt antreibbaren Bewegungseinrichtung verformt, insbesondere komprimiert, werden kann. Bevorzugt erfolgt die Verformung des elastischen Elements, an dem die zu kultivierenden Zellen anhaften derart, dass das bei einer Kompression des Elements, die mit einer Volumenverringerung einhergeht, Nährmedium aus dem Element ausgestoßen wird, während das Element in einem zumindest teilweise entspannten Zustand Nährmedium aufnimmt, insbesondere aufsaugt Die an oder in dem elastischen Element haftenden Zellen können durch ein derartiges abwechselndes Aufsaugen und Ausstoßen von Nährmedium zuverlässig und gleichmäßig mit den im Nährmedium enthaltenden Nährstoffen versorgt werden, ohne dass hierdurch eine Gasversorgung, beispielsweise mit Sauerstoff, behindert wird.

Auf oder in dem elastischen Element sind wenigstens bereichsweise zu kultivierende Zellen angeordnet, die aufgrund der Verformung des elastischen Elements auf geeignete Weise durch Bewegungsreize stimuliert werden, wodurch die Kultivierung dieser Zellen begünstigt wird. Die Verformung, insbesondere Kompression, des elastischen Elementes kann kontinuierlich in aufeinanderfolgenden Intervallen oder innerhalb variierender Zeitintervalle erfolgen. Ebenso ist es denkbar, das elastische Element gleichförmig oder mit einer veränderbaren Kraft zu beaufschlagen, so dass die Verformung des elastischen Elementes zwischen wenigstens zwei aufeinanderfolgenden Verformungsvorgängen in Art und/oder Stärke variiert Generell ist eine erfindungsgemäß ausgeführte Vorrichtung geeignet, um sowohl prokaryotische als auch eukaryotische Zellen zu kultivieren. Ebenso ist es denkbar, dass eine derartige Vorrichtung zur Herstellung von Antibiotika verwendet und/oder auf dem Gebiet der pharmakologischen Wirkstoffprüfung eingesetzt wird. Im Weitern ist eine Vorrichtung, die die Erfindung nutzt, bevorzugt zur Umsetzung einer Biofilmfermentation geeignet Es sind somit eine Vielzahl unterschiedlicher Einsatzmöglichkeiten sowie die Verwirklichung verschiedener biochemischer oder molekularbiologischer Prozesse auf der Grundlage der Nutzung der abstrakten erfinderischen Idee realisierbar.

In einer speziellen Ausführungsform der Erfindung ist vorgesehen, dass das elastische Element wenigstens teilweise schwammartiges Material aufweist In Abhängigkeit der zu kultivierenden Zellen kann es hierbei von Vorteil sein, wenn es sich bei dem schwammartigen Material um einen synthetisch hergestellten Schwamm handelt, an dessen Oberfläche und/oder in dessen Poren zumindest bereichsweise die zu kultivierenden Zellen anhaften. Vorzugsweise weist das schwammartige Material wenigstens einen Schaumstoff auf, wobei es generell denkbar ist, dass die Oberfläche des schwammartigen Materials, insbesondere des Schaumstoffs, zumindest bereichsweise oberflächenbehandelt, beispielsweise beschichtet ist.

Alternativ oder ergänzend ist es denkbar, dass das elastische Material wenigstens teilweise In-vitro-Fleisch, Bakterien, prokaryotische Zellen, wenigstens ein Polymer, eine Proteinstruktur, etwa eine Seidenproteinstruktur, Glucomannan, Zein, Kollagen, Alginat, Chitosan und/oder Zellstoff aufweist. Erfindungswesentlich hierbei ist stets, dass das elastische Element mithilfe einer gezielt bewegbaren Bewegungseinrichtung verformt, insbesondere komprimiert wird, so dass die zu kultivierenden Zellen auf geeignete Weise durch Bewegungsreize stimuliert und so zum Wachstum angeregt werden und/oder eine effektive Nährstoffversorgung der zu kultivierenden Zellen sichergestellt wird.

Gemäß einer besonderen Ausführungsform der Erfindung schließt die Bewegungseinrichtung den Gefäßinnenraum des Bioreaktors wenigstens bereichsweise gegenüber der Umgebung ab. In diesem Zusammenhang ist es denkbar, dass die Bewegungseinrichtung über zumindest einen Kolben verfügt, der bereichsweise entlang einer Innenwand des Gefäßes bewegbar angeordnet ist. Vorzugsweise ist in diesem Fall wenigstens ein Dichtelement, etwa in Form einer Ringdichtung, zwischen dem bewegbaren Kolben und der Gehäuseinnenwand vorgesehen. Denkbar ist in diesem Zusammenhang, dass ein mit dem elastischen Element verbundenes Bauteil der Bewegungseinrichtung eine Dichtfläche aufweist, die gemeinsam mit Gehäusewand ein Dichtelement einschließt, oder dass ein spezielles Antriebselement der Bewegungseinrichtung, das eine vergleichsweise kleine Dichtfläche aufweist, etwa eine mit einem Kolben verbundene Kolbenstange, zwischen sich und der Gefäßwand ein Dichtelement einschließt

Erfindungswesentlich ist wiederum, dass die Bewegungseinrichtung, beispielsweise der Kolben, mit dem elastischen Element derart gekoppelt ist, dass aufgrund der Bewegung der Bewegungseinrichtung das elastische Element verformt wird, wobei das elastische Element abwechselnd zusammengedrückt und anschließend entspannt wird. Aufgrund dieser Bewegungen werden die zumindest bereichsweise auf oder in dem elastischen Element angeordneten zu kultivierenden Zellen mit einem das Wachstum der Zellen fördernden Bewegungsreiz stimuliert Ebenso wird durch die Kompression im elastischen Element enthaltenes Nährmedium aus diesem ausgestoßen und anschließend vom entspannten elastischen Element Nährmedium aus dem Gefäßinnenraum aufgesogen.

Im Weiteren ist es von Vorteil, wenn der Gefäßinnenraum über einen Einlass und/oder einen Auslass verfügt, sodass wenigstens ein fluiddichter Strömungskanal zwischen dem Gefäßinnenraum und einer Fluidab- und/oder -zuführung oder der Umgebung geschaffen wird. Über derartige Ein- und/oder Auslässe können vorzugsweise Gase und/oder Flüssigkeiten, die zur Herstellung der für die Kultivierung der Zellen erforderlichen physiologischen Parameter im Gefäßinnenraum benötigt werden, gezielt in den Gefäßinnenraum eingeführt und wieder abgeführt werden. Vorzugsweise ist im Einlass und/oder im Auslass ein Ventil vorgesehen, das in Abhängigkeit eines Steuersignals geöffnet und geschlossen werden kann. Von besonderem Vorteil ist es, wenn ein im Einlass und/oder im Auslass angeordnetes Ventil in Abhängigkeit eines im Gefäßinnenraum herrschenden Drucks, bei dem es sich in Bezug auf eine Umgebung und/oder ein Fluidversorgungssystem um einen Über- oder einen Unterdruck handeln kann, geöffnet und geschlossen wird.

Gemäß einer ganz besonders geeigneten Weiterbildung der Erfindung verfügt ein erfindungsgemäß ausgeführter Bioreaktor sowohl über einen Einlass mit einem Einlassventil als auch einen Auslass mit einem Auslassventil, wobei sowohl das Ventil im Einlass als auch das Ventil im Auslass in Abhängigkeit eines im Gefäßinnenraum herrschenden Drucks, wobei es sich im Vergleich zu einem in einer Umgebung und/oder einem mit dem Gefäßinnenraum verbindbaren Fluidversorgungssystem um einen Über- oder einen Unterdruck handeln kann, geöffnet und geschlossen werden.

Im Weiteren ist es denkbar, dass aufgrund einer gezielten Bewegung oder einer gezielt herbeigeführten Bewegungsänderung der Bewegungseinrichtung der Druck im Innenraum derart variiert wird, dass das elastische Element zumindest lokal verformt, insbesondere komprimiert, und anschließend entspannt wird. In Abhängigkeit des sich im Gefäßinnenraum einstellenden Drucks werden das Einlass- und/oder das Auslassventil geöffnet und geschlossen, sodass eine geeignete Kultivierung der sich auf oder im elastischen Element befindenden Zellen erfolgen kann. Selbstverständlich ist es ebenfalls denkbar, wenigstens zeitweise Nährmedium über die Ventilöffnungen im Einlass und/oder Auslass bei Bedarf dem Gefäßinnenraum zuzuführen oder aus diesem abzuführen. Sowohl die Menge als auch die Zusammensetzung des im Gefäßinnenraum befindlichen Nährmediums kann auf diese Weise in Abhängigkeit der zur Kultivierung der jeweiligen im Gefäßinnenraum angeordneten Zellen erforderlichen physiologischen Parameter eingestellt werden.

Durch das Vorsehen eines Einlasses und/oder Auslasses ist es somit möglich, zumindest zeitweise einen Strömungskanal für eine Luft-, Gas- und/oder Flüssigkeitsströmung zwischen dem Gefäßinnenraum und einer außerhalb des Gefäßinnenraums angeordneten Fluidversorgung oder der Umgebung herzustellen.

In einer weiteren speziellen Ausführungsform verfügt ein Bioreaktor gemäß der Erfindung über wenigstens einen verschließbaren Zugang, über den wenigstens zeitweise eine Verbindung zum Gefäßinnenraum herstellbar ist. In diesem Zusammenhang ist es denkbar, dass über einen derartigen Zugang wenigstens zeitweise ein die Gefäßwand durchdringender Strömungskanal freigegeben wird, über den wenigstens ein Medium, das wenigstens einen Nährstoff, Bakterien und/oder ein Fluid aufweist, in den Gefäßinnenraum eingeleitet oder aus diesem abgeführt wird.

Gemäß einer besonderen Weiterbildung der Erfindung handelt es sich bei einem speziell ausgeführten Zugang um einen Probenahmezugang, über den aus dem Gefäßinnenraum zumindest ein Teil der darin befindlichen Zellen und/oder des Nährmediums entnehmbar ist. In diesem Zusammenhang ist es denkbar, dass zumindest zeitweise, bevorzugt automatisiert, eine Probe des im Gefäßinnenraum angeordneten Nährmediums und/oder Zellen entnommen und untersucht wird. In Abhängigkeit der Untersuchungsergebnisse zumindest wird vorzugsweise wenigstens ein Parameter, der Einfluss auf die Kultivierung der Zellen hat, bedarfsgerecht variiert. In diesem Zusammenhang ist es denkbar, dass in Abhängigkeit eines Untersuchungsergebnisses entweder die Menge oder Zusammensetzung des im Gefäßinnenraum befindlichen Nährmediums verändert oder der Bewegungsvorgang der Bewegungseinrichtung und damit des bewegten elastischen Elements gezielt verändert wird.

Im Übrigen kann alternativ oder ergänzend zum Probenahmezugang wenigstens ein Zugang vorgesehen sein, der zur wenigstens bereichsweisen Anordnung eines Sensorelementments, etwa einer Sonde zur Erfassung eines pH-Werts oder einer O₂-Konzentration, im Bereich des Gefäßinnenraums vorgesehen ist.

Um eine geeignete Bewegung der Bewegungseinrichtung und somit Verformung des elastischen Elements zu bewirken, ist die Bewegungseinrichtung an wenigstens eine geeignete Antriebseinheit ankoppelbar, die wiederum mithilfe einer zentralen Steuereinheit angesteuert wird. Es ist denkbar, dass die Antriebseinheit wenigstens ein mechanisch, pneumatisch, hydraulisch, elektrisch oder elektropneumatisch angetriebenes Antriebselement aufweist, das zur Erzeugung einer Bewegung auf die Bewegungseinrichtung einwirkt.

Gemäß einer besonderen Ausführungsform der Erfindung verfügt die Bewegungseinrichtung über wenigstens einen Kolben, der mithilfe einer elektrischen, pneumatischen oder elektropneumatischen Antriebseinheit in die für die zeitweise Verformung des elastischen Elements benötigte Bewegung versetzt wird. Der Kolben wird hierbei derart bewegt, dass das im Gefäßinnenraum angeordnete elastische Element abwechselnd komprimiert und entspannt wird, sodass die auf oder in dem elastischen Element angeordnet Zellen aufgrund der Bewegung stimuliert und zu einem speziellen Wachstum angeregt werden. Durch diese Einleitung von Bewegungsreizen wird die Kultivierung der Zellen, beispielsweise die Erzeugung eines Zellverbundes, auf vergleichsweise einfache und störunanfällige Weise begünstigt.

Ein erfindungsgemäß ausgeführter Bioreaktor verfügt im Weiteren vorzugsweise über wenigstens ein Sensorelement, das zumindest einen Parameter, insbesondere einen physiologischen Parameter, im Innenraum des Gefäßes erfasst. Hierbei kann es sich sowohl um die Konzentration des Nährmediums, eine Zusammensetzung des Nährmediums, eine Temperatur, einen Druck, eine Gaskonzentration und/oder einen sonstigen physiologischen Parameter handeln, der Einfluss auf die Kultivierung der im Gefäßinnenraum auf oder in dem elastischen Element angeordneten Zellen hat In Abhängigkeit eines erfassten Messwerts und eines relevanten Sollwerts ist denkbar, dass eine zentrale Steuereinheit ein Steuersignal erzeugt, welches zur Ansteuerung einer Antriebseinheit zur Initiierung der Bewegung der Bewegungseinrichtung, einer Fluidzu- und -abführung und/oder einer Dosiereinheit erzeugt. Auf diese Weise ist es möglich, eine Menge, eine Zusammensetzung und/oder eine Konzentration des im Gefäßinnenraum befindlichen Nährmediums, seiner Komponenten und/oder eines sonstigen Stoffes gezielt zu verändern. Die Steuereinheit generiert geeignete Steuersignale hierbei bevorzugt in Abhängigkeit eines Vergleichs zwischen dem von wenigstens einem Sensorelement erfassten Messwert und einem Sollwert, der beispielsweise in einer Datenbank abgelegt ist

Gemäß einer ganz speziellen Ausführungsform ist vorgesehen, dass die Antriebseinheit zumindest eine Nocke aufweist, die wiederum während ihrer Bewegung in Wirkverbindung mit der Bewegungseinrichtung steht und eine Bewegung initiiert, die zu einer wechselweisen Komprimierung sowie Entspannung des elastischen Elements führt. Die wenigstens eine Nocke, die etwa Teil einer Welle sein kann, führt vorzugsweise eine Drehbewegung aus, und indiziert so eine Bewegung der zumindest teilweise im Gefäßinnenraum angeordneten Bewegungseinrichtung, etwa eines Kolbens, in unterschiedlicher Richtung. Gemäß einer besonderen Weiterbildung der Erfindung ist es diesbezüglich denkbar, dass eine Mehrzahl von Bioreaktoren auf geeignete Weise nebeneinander angeordnet sind und die Bewegungseinrichtungen der einzelnen Bioreaktoren mithilfe einer Nockenwelle, die über eine Mehrzahl von Nocken verfügt, angetrieben werden.

Auf vorteilhafte Weise ist zwischen einer Bewegungseinrichtung, die zumindest zeitweise Verformung des elastischen Elements bewirkt und einer Innenwand des Gefäßes wenigstens ein Dichtelement vorgesehen, durch das der Gefäßinnenraum fluiddicht, also flüssigkeits- und/oder gasdicht gegenüber der Umgebung abgedichtet ist Ein derartiges Dichtelement kann beispielsweise in Form einer Ringdichtung, die die Bewegungseinrichtung, etwa einen Kolben der Bewegungseinrichtung, umgibt, ausgeführt sein. Generell ist es denkbar, dass das wenigstens eine Dichtelement gemeinsam mit der Bewegungseinrichtung bewegt wird oder aber eine Relativbewegung zwischen Dichtelement und Bewegungseinrichtung erfolgt. Bei der letztgenannten Ausführungsform ist das wenigstens eine Dichtelement vorzugsweise mit Hilfe geeigneter Fixierungselemente zumindest abschnittsweise einem Bereich der Gefäßinnenwand fixiert.

Gemäß einer weiteren speziellen Ausführungsform der Erfindung ist denkbar, dass die Bewegungseinrichtung selbst über einen Einlass und/oder einen Auslass verfügt, der zumindest den Teil eines Strömungskanals zwischen dem Gefäßinnenraum und einer Fluidversorgung oder der Umgebung bildet. In diesem Fall befinden sich somit der Einlass und/oder der Auslass nicht in einer Wand des Gefäßes, in dessen Innenraum Zellen kultiviert werden, sondern innerhalb der Bewegungseinrichtung. Zum Beispiel ist es denkbar, dass ein relativ zur Gefäßwand bewegbar gelagerter Kolben, über wenigstens einen derartigen Einlass und/oder Auslass verfügt, in dem wiederum auf vorteilhafte Weise ein Ventil zum gezielten Öffnen und Schließen des Einlasses und/oder des Auslasses angeordnet ist.

Eine weitere spezielle Weiterbildung der Erfindung sieht vor, dass wenigstens ein Fixierungsmittel vorgesehen ist, das zumindest bereichsweise das elastische Element innerhalb des Gefäßes fixiert. Ein derartiges Fixierungsmittel kann beispielsweise wenigstens ein Hakenelement aufweisen, das derart in das elastische Element eingreift, dass eine Bewegung des elastischen Elements relativ zum Innenraum und/oder zur Bewegungseinrichtung zumindest in eine Richtung begrenzt oder sogar verhindert. Generell ist es denkbar, dass das wenigstens eine Fixierungsmittel an einer Innenwand des Gefäßes und/oder an der Bewegungsrichtung an einer dem Gefäßinnenraum zugewandten Seite angeordnet ist. Mithilfe eines derartigen Fixierungsmittels oder einer Mehrzahl von Fixierungsmittels, etwa in Form von Haken, Dornen oder Anschlägen kann sichergestellt werden, dass das elastische Element aufgrund einer Bewegung der Bewegungseinrichtung gezielt in wenigstens eine bestimmte Richtung bewegt wird und/oder dass Bewegungen in zumindest eine ausgewählte Richtung verhindert werden. Durch geeignete Auswahl und Anordnung entsprechender Fixierungsmittel wird sichergestellt, dass gezielte Bewegungen eines elastischen Elements, das mithilfe einer Bewegungseinrichtung bewegt wird, im Inneren des Gefäßes erzeugt und die zu kultivierenden Zellen auf geeignete Weise stimuliert werden, insbesondere um verbesserte Wachstumsbedingungen herzustellen.

Im Folgenden wird die Erfindung ohne Beschränkung des allgemeinen Erfindungsgedankens anhand von Ausführungsbeispiele unter Bezugnahme auf die Figuren näher erläutert.

Dabei zeigen:
- Fig. 1:: Erfindungsgemäß ausgeführter Bioreaktor in einer Betriebssituation mit entspanntem elastischem Element,
- Fig. 2:: Erfindungsgemäß ausgeführter Bioreaktor in einer Betriebssituation mit komprimiertem elastischem Element sowie
- Fig. 3:: Schematische Darstellung eines erfindungsgemäß ausgeführten Bioreaktors, dessen Bewegungseinrichtung über einen Kolben und eine Kolbenstange verfügt.

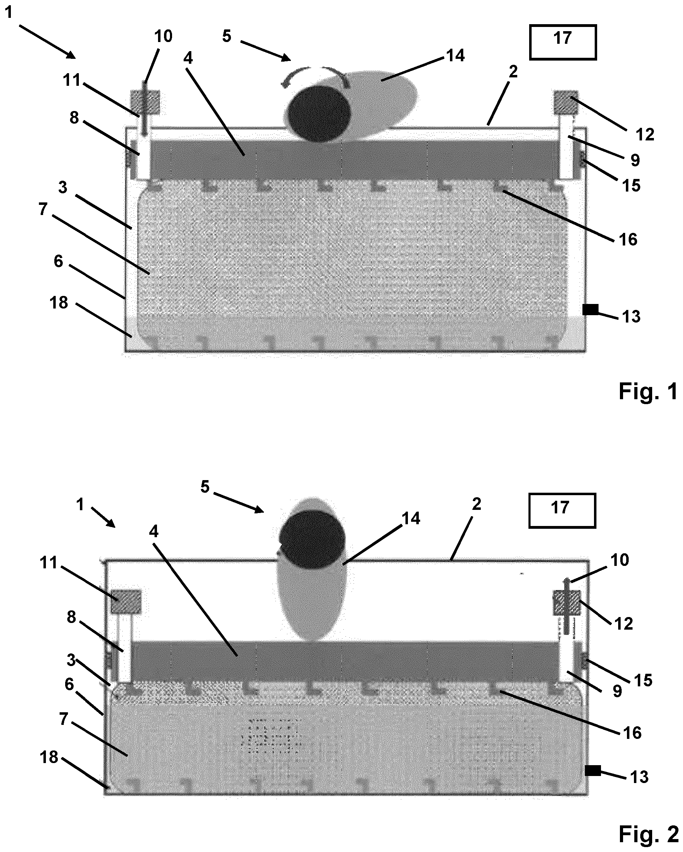

Die Fig. 1 zeigt einen erfindungsgemäß ausgeführten Bioreaktor 1 mit einem sterilisierbaren Gefäß 2, in dessen Gefäßinnenraum 3 ein elastisches Element 7 in Form eines Schwamms und eine relativ zur Gefäßwand 6 bewegbar gelagerte Bewegungseinrichtung 4 angeordnet sind. Das elastische Element 7 ist im Gefäßinnenraum mithilfe von Haken, die als Fixierungselemente 16 dienen, fixiert. Auf der Oberfläche und in den Poren des elastischen Elementes 7 sind Zellen angeordnet, die auf geeignete Weise kultiviert werden, sodass großflächige oder sogar dreidimensionale Zellstrukturen, beispielsweise Muskelfasern oder künstliches Fleisch, erzeugt werden können.

Wesentlich für den in Fig. 1 gezeigten Bioreaktor 1 ist, dass die Bewegungseinrichtung 4, die gemäß dem erläuterten Ausführungsbeispiel über einen entlang einer Innenseite der Gefäßwand 6 bewegbaren Kolben verfügt, der während eines Betriebs das elastische Element 7 im Gefäßinnenraum 3 mit den daran anhaftenden Zellen zeitweise verformt, wobei das elastische Element abwechselnd komprimiert und entspannt wird, was zu einer entsprechenden Veränderung des Volumens führt. Der Kolben der Bewegungseinrichtung 4 bewegt sich hierfür wechselweise in entgegengesetzter Richtung und zwar entweder in das Gefäß 2 hinein oder aus dem Gefäß heraus, sodass es auf geeignete Weise abwechselnd zu einer Kompression und Entspannung des elastischen Elementes 7 und damit zu einer Stimulierung der im an dem elastischen Element anhaftenden Zellen kommt, wodurch ein verbessertes Zellwachstum realisierbar ist. Ein weiterer Vorteil besteht darin, dass das elastische Element 7, das in diesem Fall schwammartiges Material aufweist, abwechselnd Nährmedium 18 aufnimmt und wieder abgibt, sodass die Zellen gleichmäßig mit den im Nährmedium 18 enthaltenen Nährstoffen versorgt werden und trotzdem ausreichend mit der im Gefäßinnenraum enthaltenen Gasatmosphäre, insbesondere mit dem darin enthaltenen Sauerstoff und oder CO₂, in Kontakt kommen.

Zwischen dem Kolben der Bewegungseinrichtung 4 und einer Innenseite der seitlichen Gefäßwände 6 ist ferner ein Dichtelement 15, vorgesehen, das eine sichere Abdichtung des Gefäßinnenraums 3 gegenüber der Umgebung gewährleistet Gemäß dem hier gezeigten Ausführungsbeispiel ist das Dichtelement 15 in Form eines Dichtrings ausgeführt und an dem Kolben der Bewegungseinrichtung 4 befestigt, sodass es gemeinsam mit diesem bewegt wird.

Die Bewegungseinrichtung 4 steht auf einer dem Gefäßinnenraum 3 entgegengesetzten Seite mit einer Antriebseinheit 5 in Wirkverbindung, die als Antriebselement 14 eine drehbar gelagerte und von einem Elektromotor angetriebene Nocke verfügt. Durch eine Drehung der Nocke wird der Kolben der Bewegungseinrichtung 4 abwechselnd entweder in Richtung des Gefäßinnenraums oder in Richtung der Nocke bewegt, wobei während der Bewegung in Richtung des Gefäßinnenraums 3 das elastische Element 7 mit den daran anhaftenden Zellen komprimiert wird. Dieser Betriebszustand, in dem das elastische Element 7 komprimiert ist, ist in Fig. 2 gezeigt

Nicht nur an der den Boden des Gefäßes 2 bildenden Gefäßwand 6, sondern auch auf der dem Gefäßinnenraum 3 und damit dem elastischen Element 7 zugewandten Seite des Kolbens der Bewegungseinrichtung 4 sind als Fixierungselemente 16 Haken angeordnet. Mit diesen hakenförmigen Fixierungselementen 16 wird das elastische Element in seiner Position im Gefäßinnenraum 3 fixiert und beispielsweise ein ungewolltes Verrutschen zuverlässig verhindert.

Im Weiteren sind im Bereich des Kolbens der Bewegungseinrichtung 4 ein Einlass 8 mit einem Einlassventil 11 sowie ein Auslass 9 mit einem Auslassventil 12 vorgesehen. Über den Einlass kann aus einer entsprechenden Fluidversorgung 10 Gas und/oder Nährmedium 18 in den Gefäßinnenraum 3 eingeleitet und entsprechend über den Auslass 9 abgeführt werden. Ein Öffnen und Schließen der im Einlass 8 und im Auslass 9 angeordneten Ventile 11, 12 erfolgt hierbei in Abhängigkeit des im Gefäßinnenraum 3 herrschenden Drucks. Eine derartige Steuerung der Ventile 11, 12 hat den großen Vorteil, dass ein Öffnen und Schließen der Ventile 11, 12 vergleichsweise einfach und sicher realisiert werden kann. Alternativ oder in Ergänzung ist es denkbar, eine zentrale Steuereinheit 17 vorzusehen, die geeignete Steuersignale zur Umsetzung eines effektiven Kultivierungsprozesses erzeugt und Signale uni- oder bidirektional mit den Ein- und Auslassventilen 11, 12, aber auch mit einer Antriebseinheit 5, wenigstens einem Sensorelement, einer Fluidversorgung 10 und/oder einer Einrichtung zur gezielten Temperierung des Gefäßinnenraums 3 austauscht Eine entsprechende Signal- und/oder Datenübertragung kann drahtgebunden oder drahtlos erfolgen.

Fig. 1 zeigt einen erfindungsgemäß ausgeführten Bioreaktor 1 in einem Betriebszustand, in dem sich der Kolben der Bewegungseinrichtung 4 in einem aus dem Gefäßinnenraum 3 herausbewegten Zustand befindet, sodass das elastische Element 7 entspannt ist und zumindest teilweise Nährmedium 18 aufnehmen kann.

Demgegenüber zeigt Fig. 2 einen erfindungsgemäß ausgeführten Bioreaktor 1 in einem Betriebszustand, in dem sich der Kolben der Bewegungseinrichtung 4 in einem maximal in den Gefäßinnenraum 3 eingefahrenen Zustand befindet, sodass das elastische Element 7 auf ein minimales Volumen komprimiert ist Die als Antriebselement 14 der Antriebseinheit 5 verwendete Nocke ist hierbei im Vergleich zu dem in Fig. 1 gezeigten Betriebszustand entgegen dem Uhrzeigersinn um etwa 260° nach links verdreht, wodurch eine entsprechende Bewegung des Kolbens initiiert wurde.

Durch gezielte Variation der Form einer Nocke und/oder Veränderung der Drehgeschwindigkeit können sowohl der Grad der Verformung als auch die Geschwindigkeit, mit der das im Gefäßinnenraum 3 angeordnete elastische Element 7 verformt wird, verändert werden. Sowohl die Geschwindigkeit einer Formänderung als auch der Grad einer Formänderung des elastischen Elementes 7 erfolgt hierbei in Abhängigkeit der Parameter, die für eine optimale Kultivierung der im Gefäßinnenraum 3 und im oder am elastischen Element 7 angeordnet Zellen benötigt werden.

Mithilfe des in den Figuren 1 und 2 gezeigten Bioreaktors 1 ist es auf vergleichsweise einfache Weise möglich, bei der Kultivierung von Zellen prokaryotischen oder eukaryotischen Ursprungs für den jeweiligen Zelltyp angepasste physiologische Wachstumsparameter einzustellen. Hierbei können insbesondere die Art und Größe der Stimuli, die die Zellen zum Wachstum anregen sollen, variiert werden. Ein erfindungsgemäß ausgeführter Bioreaktor 1 ermöglicht die Anzucht spezieller Zellverbände aufgrund der Applikation externer Stimuli im größeren Maßstab. Ferner lassen sich sogar dreidimensionale Gewebeverbände für die Medizin oder Muskelfasern als Ausgangsmaterial für kultiviertes Fleisch herstellen. Ein erfindungsgemäß ausgeführter Bioreaktor verfügt hierbei über die drei in den Figuren 1 und 2 gezeigten Hauptkomponenten, nämlich ein sterilisierbares Gefäß 2, eine Bewegungseinrichtung 4 mit einem Kolben, ein wechselweise komprimiertes und entspanntes elastisches Element 7 sowie eine Fluidversorgung mit einem Einlass 8 und einem Auslass 9 für den benötigten Gasaustausch.

Des Weiteren ist ein Zugang 13 vorgesehen, der gemäß dem dargestellten Ausführungsbeispiel vorgesehen ist, um bedarfsgerecht Proben des Nährmediums 18 und/oder der im Gefäßinnenraum 3 angeordneten Zellen zu entnehmen.

Das elastische Element 7 ist gemäß der hier beschriebenen speziellen Ausführungsform der Erfindung als Schwamm aus Glucomannan ausgebildet In Abhängigkeit der jeweils zu kultivierenden Zellen kann das elastische Element allerdings auch andere Materialien aufweisen, die geeignet sind, als Träger für die zu kultivierenden Zellen zu dienen, und die zumindest teilweise Nährmedium 18 aufnehmen können. Mithilfe wenigstens eines geeigneten Fixierungselements lässt sich das elastische Element 7 auf vorteilhafte Weise im Gefäßinnenraum 3 fixieren. Hierzu sehen die in den Figuren 1 und 2 gezeigten Ausgestaltungen vor, dass am Boden des Bioreaktors 1 und an der Unterseite des Kolbens der Bewegungseinrichtung 4 Haken zur Verankerung angeordnet sind. Das elastische Element 7 dient jeweils als Strukturmatrix für die Anhaftung der zu kultivierenden Zellen.

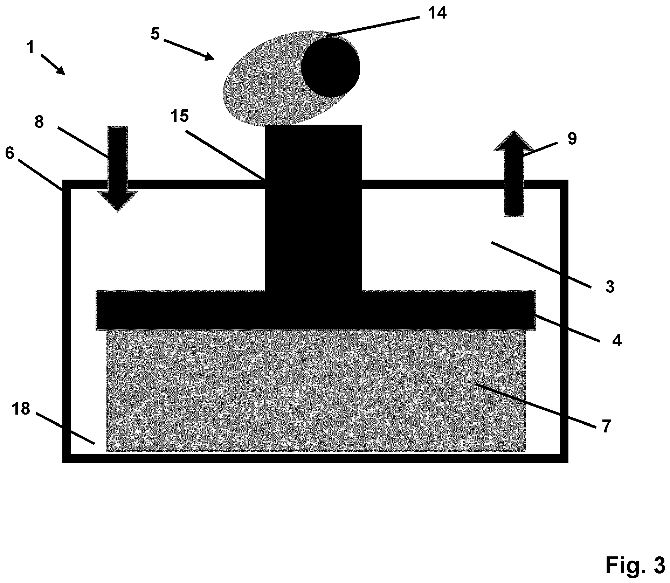

Ergänzend zeigt Fig. 3 eine schematische Darstellung eines erfindungsgemäß ausgeführten Bioreaktors 1, dessen Bewegungseinrichtung 1 und Fluidversorgung allerdings abweichend zu dem in den Figuren 1 und 2 gezeigten Bioreaktor 1 ausgeführt ist Das erfindungswesentliche Funktionsprinzip, nämlich die Kompression eines im Gefäßinnenraum 3 angeordneten elastischen Elements 7 gemeinsam mit den daran anhaftenden zu kultivierenden Zellen bewirkt durch eine Bewegungseinrichtung 4, wird auch mit der in Fig. 3 gezeigten Vorrichtung verwirklicht. Der wesentliche Unterschied zu dem in den Figuren 1 und 2 gezeigten Bioreaktor 1 besteht darin, dass die Bewegungseinrichtung 4 einen Kolben und eine Kolbenstange, die die Verbindung zwischen dem auf das elastische Element 7 einwirkenden Kolben im Gefäßinnenraum 3 und einer außerhalb des Gefäßes 2 angeordneten Antriebseinheit 5 herstellt. Gemäß dieser Ausführungsform durchragt die Kolbenstange die Gehäusewand 6, sodass ein vergleichsweise kleines Dichtelement 15 zwischen den Dichtflächen der Gefäßwand 15 und der Kolbenstange angeordnet ist. Die Zu- und Abfuhr wenigstens eines Fluids in den und/oder aus dem Gefäßinnenraum 3 erfolgt in diesem Fall über einen Einlass 8 und einen Auslass 9, die innerhalb der Gefäßwand 6 angeordnet sind und so eine Verbindung zwischen dem Gefäßinnenraum 3 oder einem Fluidversorgungssystem (in dieser Ansicht nicht dargestellt) herstellen. Die Übrigen Bauelemente, wie etwa unterschiedliche Zugänge oder eine Steuereinheit, wie sie in den Figuren 1 und 2 gezeigt sind, sind der schematischen Darstellung gemäß Fig. 3 nicht zu entnehmen, können aber ohne Beschränkung des allgemeinen Erfindungsgedankens auch bei dieser speziellen Ausführungsform zum Einsatz kommen.

Die Bewegung der Bewegungseinrichtung 4 und damit des elastischen Elements 7 im Gefäßinnenraum 3 wird mithilfe einer geeigneten Antriebseinheit 5 erzeugt. In den Figuren verfügt die Antriebseinheit 5 hierfür über eine Nocke als Antriebselement 14, die Teil einer Nockenwelle sein kann und aufgrund ihrer Bewegung eine Auf- und Abbewegung des Kolbens initiiert. Selbstverständlich ist es denkbar, die Erfindung auch mit Hilfe anderer Antriebseinheiten, Antriebselemente und/oder Bewegungseinrichtungen zu verwirklichen als denjenigen, die in den Figuren gezeigt sind, zu verwirklichen. Wesentlich für die Erfindung ist stets, dass im Inneren des Gefäßes des Bioreaktors ein elastisches Element mit daran anhaftenden Zellen angeordnet ist, das mithilfe einer Bewegungseinrichtung wechselweise verformt, insbesondere komprimiert und entspannt wird. Auf vorteilhafte Weise wird für ein derartiges elastisches Element ein schwammartiges Material, das synthetischen oder natürlichen Ursprung haben kann, verwendet. Die physikalisch induzierte Reizung der zu kultivierenden Zellen erfolgt hierbei durch die mechanische Stauchung und Entspannung des elastischen Elementes, das die zellbehaftete Matrix bildet. Auf diese Weise wird nicht nur die erforderliche Gasversorgung, insbesondere der Luftaustausch über die im Einlass und Auslass angeordneten Ventile, sondern gleichzeitig eine konstante, homogene Versorgung mit Nährstoffen durch das Nährmedium, das abwechselnd vom elastischen Element aufgenommen und abgegeben wird, realisiert

Ein erfindungsgemäß ausgeführter Bioreaktor stellt somit eine einfache und effiziente technische Lösung zur Stimulierung von Zellen und Biofilmen für die gewünschte phänotypische Transformation, beispielsweise zur Differenzierung von Myoblasten zu Mikrotubuli, dar. Im Weiteren ist ein derartiger Bioreaktor leicht skalierbar, verfügt über vergleichsweise verschleißarme Bauelemente und ermöglicht eine besonders schonende Zellkultivierung.

### Bezugszeichenliste

- 1: Bioreaktor
- 2: Gefäß
- 3: Gefäßinnenraum
- 4: Bewegungseinrichtung
- 5: Antriebseinheit
- 6: Gefäßwand
- 7: elastisches Element
- 8: Einlass
- 9: Auslass
- 10: Fluidversorgung
- 11: Einlassventil
- 12: Auslassventil
- 13: Zugang
- 14: Antriebselement
- 15: Dichtelement
- 16: Fixierungselement
- 17: Steuereinheit
- 18: Nährmedium

## Patentansprüche

1. Bioreaktor (1) zur Kultivierung von Zellen mit wenigstens einem Gefäß (2), das eingerichtet ist, um in einem wenigstens teilweise von Wänden (6) des Gefäßes (2) gegenüber einer Umgebung abgeschlossenen Gefäßinnenraum (3) die zu kultivierenden Zellen und zumindest ein Nährmedium aufzunehmen, und mit einer Bewegungseinrichtung (4), die mit einer Antriebseinheit (5) verbindbar ist und die die zu kultivierenden Zellen wenigstens zeitweise bewegt oder mit einer Kraft beaufschlagt,
**dadurch gekennzeichnet, dass** in dem Gefäßinnenraum (3) ein elastisches Element (7) angeordnet ist, das derart ausgeführt ist, dass zumindest in einem Bereich des elastischen Elements wenigstens ein Teil des Nährmediums aufnehmbar ist und dass die zu kultivierenden Zellen wenigstens bereichsweise in und/oder an dem elastischen Element zumindest zeitweise haften und dass die Bewegungseinrichtung (4) eingerichtet ist, um das elastische Element (7) wenigstens zeitweise zu verformen.

2. Bioreaktor nach Anspruch 1,
**dadurch gekennzeichnet, dass** das elastische Element (7) schwammartiges Material aufweist.

3. Bioreaktor nach Anspruch 2,
dass das schwammartige Material einen Schaumstoff aufweist.

4. Bioreaktor nach einem der vorangehenden Ansprüche,
**dadurch gekennzeichnet, dass** das elastische Element (7) In-vitro-Fleisch, Bakterien, wenigstens ein Polymer, eine Proteinstruktur, Glucomannan, Zein, Kollagen, Alginat, Chitosan und/oder Zellstoff aufweist

5. Bioreaktor nach einem der vorangehenden Ansprüche,
**dadurch gekennzeichnet, dass** die Bewegungseinrichtung (4) den Gefäßinnenraum (3) wenigstens bereichsweise gegenüber der Umgebung abschließt

6. Bioreaktor nach einem der vorangehenden Ansprüche,
**dadurch gekennzeichnet, dass** jeweils wenigstens ein verschließbarer Einlass (8) und Auslass (9) vorgesehen sind, durch die zumindest zeitweise ein Strömungskanal für eine Luft- und/oder Gasströmung zwischen dem Gefäßinnenraum (3) und einer Fluidversorgung (10) oder der Umgebung herstellbar ist

7. Bioreaktor nach Anspruch 6,
**dadurch gekennzeichnet, dass** im Einlass (8) ein Einlassventil (11) und im Auslass (9) ein Auslassventil (12) angeordnet und derart ausgeführt sind, dass ein Öffnen und Schließen des Einlass- (11) und/oder des Auslassventils (12) durch eine Druckänderung im Gefäßinnenraum (3) bewirkt wird.

8. Bioreaktor nach einem der vorangehenden Ansprüche,
**dadurch gekennzeichnet, dass** das Gefäß (2) wenigstens einen verschließbaren Zugang (13) aufweist, über den zumindest zeitweise eine Verbindung von außerhalb des Gefäßes (2) zum Gefäßinnenraum (3) herstellbar ist.

9. Bioreaktor nach einem der vorangehenden Ansprüche,
**dadurch gekennzeichnet, dass** die Antriebseinheit (5) wenigstens ein mechanisch, pneumatisch, hydraulisch, elektrisch oder elektropneumatisch angetriebenes Antriebselement (14) aufweist, das zur Erzeugung einer Bewegung auf die Bewegungseinrichtung (4) einwirkt.

10. Bioreaktor nach einem der vorangehenden Ansprüche,
**dadurch gekennzeichnet, dass** die Bewegungseinrichtung (4) über zumindest einen im oder am Gefäß (2) bewegbar gelagerten Kolben verfügt, bei dessen Bewegung das elastische Element (7) in aufeinanderfolgenden Zeitintervallen wenigstens bereichsweise komprimiert und entspannt wird.

11. Bioreaktor nach Anspruch 10,
**dadurch gekennzeichnet, dass** die Antriebseinheit (5) wenigstens eine drehbar gelagerte Nocke als Antriebselement (14) aufweist, das die Bewegung des Kolbens initiiert.

12. Bioreaktor nach einem der vorangehenden Ansprüche,
**dadurch gekennzeichnet, dass** die Bewegungseinrichtung (4) über wenigstens eine im oder am Gefäß (2) bewegbar gelagerte Membran und/oder Schraube verfügt, bei deren Bewegung das elastische Element (7) in aufeinanderfolgenden Zeitintervallen wenigstens bereichsweise komprimiert und entspannt wird.

13. Bioreaktor nach einem der vorangehenden Ansprüche,
**dadurch gekennzeichnet, dass** wenigstens bereichsweise zwischen der Bewegungseinrichtung (4) und dem Gefäß (2) ein Dichtelement (15) angeordnet ist, das den Gefäßinnenraum (3) gegenüber der Umgebung flüssigkeits- und/oder gasdicht abdichtet.

14. Bioreaktor nach einem der vorangehenden Ansprüche,
**dadurch gekennzeichnet, dass** das elastische Element (7) wenigstens teilweise oberflächenbehandelt ist

15. Bioreaktor nach einem der vorangehenden Ansprüche,
**dadurch gekennzeichnet, dass** wenigstens ein Fixierungselement (16) zur zumindest bereichsweisen Fixierung des elastischen Elements (7) an einer dem Gefäßinnenraum zugewandten Innenseite wenigstens einer Gefäßwand (6) und/oder an der Bewegungseinrichtung (4) vorgesehen ist.
